# EUROPEAN PATENT SPECIFICATION

(11) **EP 3 316 792 B1**
(45) Date of publication and mention of the grant of the patent: **22.05.2019**
(21) Application number: 16733531.4
(22) Date of filing: 29.06.2016
(51) Int. Cl.: G01S 15/89, G10K 11/02, A61B 8/12, A61B 8/00, B06B 1/06, G01S 7/52

(54) **INTRAVASCULAR ULTRASOUND DEVICE WITH IMPEDANCE MATCHING STRUCTURE**
INTRAVASKULÄRE ULTRASCHALLVORRICHTUNG MIT IMPEDANZANPASSUNGSSTRUKTUR
DISPOSITIF À ULTRASONS INTRAVASCULAIRE À STRUCTURE D'ADAPTATION D'IMPÉDANCE

(30) Priority: 30.06.2015 US 201562187069 P
(43) Date of publication of application: 09.05.2018
(73) Proprietor: Koninklijke Philips N.V., 5656 AE Eindhoven (NL); Volcano Corporation, San Diego, CA 92130 (US)
(72) Inventor: ZHAO, Shukui, 5656 AE Eindhoven (NL); CORL, Paul, Douglas, 5656 AE Eindhoven (NL)
(74) Representative: Steffen, Thomas
(86) International application number: PCT/EP2016/065217
(87) International publication number: WO 2017/001525

(56) References cited:
- US-A1- 2014 187 960
- US-B1- 7 226 417

## Description

### TECHNICAL FIELD

The present disclosure relates generally to intravascular ultrasound (IVUS) imaging systems and, in particular, to impedance matching structures for intravascular ultrasound devices. Such impedance matching structures may provide acoustic transition from a generally rigid ultrasound transducer to a lower-impedance environment. For example, some embodiments of the present disclosure provide an IVUS imaging system with an impedance matching structure configured to provide a transition particularly suited to imaging within a human blood vessel.

### BACKGROUND

Minimally invasive sensing systems are routinely utilized by medical professionals to evaluate, measure, and diagnose conditions within the human body. As one example, intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device includes one or more ultrasound transducers arranged at a distal end of an elongate member. The elongate member is passed into the vessel thereby guiding the transducers to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

There are two general types of IVUS devices in use today: rotational and solid-state (also known as synthetic aperture phased array). For a typical rotational IVUS device, a single ultrasound transducer element is located at the tip of a flexible driveshaft that spins inside a plastic sheath inserted into the vessel of interest. In side-looking rotational devices, the transducer element is oriented such that the ultrasound beam propagates generally perpendicular to the longitudinal axis of the device. In forward-looking rotational devices, the transducer element is pitched towards the distal tip so that the ultrasound beam propagates more towards the tip (in some devices, being emitted parallel to the longitudinal centerline). The fluid-filled sheath protects the vessel tissue from the spinning transducer and driveshaft while permitting ultrasound signals to propagate from the transducer into the tissue and back. As the driveshaft rotates, the transducer is periodically excited with a high voltage pulse to emit a short burst of ultrasound. The same transducer then listens for the returning echoes reflected from various tissue structures. The IVUS medical sensing system assembles a two dimensional display of the tissue, vessel, heart structure, etc. from a sequence of pulse/acquisition cycles occurring during a single revolution of the transducer. In order to image a length of a vessel, the transducer element is drawn through the vessel as it spins.

In contrast, solid-state IVUS devices utilize a scanner assembly that includes an array of ultrasound transducers connected to a set of transducer controllers. In side-looking and some forward-looking IVUS devices, the transducers are distributed around the circumference of the device. In other forward-looking IVUS devices, the transducers are arranged in a linear array at the distal tip and pitched so that the ultrasound beam propagates closer to parallel with the longitudinal centerline. The transducer controllers select transducer sets for transmitting an ultrasound pulse and for receiving the echo signal. By stepping through a sequence of transmit-receive sets, the solid-state IVUS system can synthesize the effect of a mechanically scanned transducer element but without moving parts. Since there is no rotating mechanical element, the transducer array can be placed in direct contact with the blood and vessel tissue with minimal risk of vessel trauma. Furthermore, because there is no rotating element, the interface is simplified. The solid-state scanner can be wired directly to the medical sensing system with a simple electrical cable and a standard detachable electrical connector. While the transducers of the scanner assembly do not spin, operation is similar to that of a rotational system in that, in order to image a length of a vessel, the scanner assembly is drawn through the vessel while stepping through the transmit-receive sets to produce a series of radial scans.

Both types of imaging devices utilize ultrasound reflections produced by structures in the surrounding environment to create an image. However, the same physical properties that produce echoes at tissue and fluid boundaries also produce undesirable echoes within the elongate member. These echoes are due in part to the different acoustical impedances of the various materials used to make the elongate member. A select number of these echoes are beneficial. For example, some backside reflections constructively interfere with the forward beam thereby increasing power. However, most echoes produced by the elongate member merely contribute noise. This noise makes environmental reflections more difficult to discern, thereby reducing resolution, sensitivity, and fidelity. Accordingly, there remains a need for intravascular devices and systems with improved acoustical properties that enhance IVUS image quality.

### SUMMARY

Some embodiments of the present disclosure are directed to an intravascular device having an impedance matching structure coupled to an ultrasound transducer that provides an acoustic impedance selected to reduce reflections internal to the intravascular device.

In one exemplary implementation, an ultrasound imaging device is provided. The ultrasound imaging device includes a flexible elongate member; and an ultrasound scanner assembly disposed at a distal portion of the flexible elongate member. The ultrasound scanner assembly includes a plurality of ultrasound transducers arranged circumferentially, and an impedance matching structure is coupled to the plurality of ultrasound transducers. In some such embodiments, the impedance matching structure is disposed radially outward from the plurality of ultrasound transducers and includes a polymer film and a conductor layer coupled to the polymer film. In some such embodiments, the conductor layer is disposed radially inward on the polymer film, and in some such embodiments, the conductor layer is disposed radially outward on the polymer film. The impedance matching structure may be disposed radially inward from the plurality of ultrasound transducers and includes a backing material that includes an epoxy containing at least one of: a ceramic material or a metal.

In one exemplary implementation, an imaging device is provided. The imaging device includes a flexible elongate member; an ultrasound transducer disposed at a distal portion of the flexible elongate member and oriented to produce an ultrasound emission in a first direction; and a plurality of layers disposed on the ultrasound transducer in the first direction and configured to produce an acoustic impedance matching between the ultrasound transducer and an environment of the ultrasound transducer. In some such embodiments, the plurality of layers includes a polymer film layer and a conductor layer coupled to the polymer film layer. In some such embodiments, the conductor layer has a thickness in a radial direction between about 0.5 µm and about 2.0 µm. In some such embodiments, the conductor layer has a thickness in a radial direction between about 0.5 µm and about 1.0 µm.

In one exemplary implementation, an imaging catheter configured for insertion into the body is provided. The imaging catheter includes a catheter body having a proximal portion and a distal portion; a sensor assembly mounted on the distal portion and that includes: an ultrasound transducer configured to transmit an ultrasound signal in a first direction, a first acoustic structure disposed on the ultrasound transducer in the first direction, and a second acoustic structure disposed on the ultrasound transducer in a second direction opposite the first direction. In some such embodiments, the first acoustic structure includes a metal-containing layer disposed on a polymer layer, and the second acoustic structure includes a backing material. The backing material may have a thickness selected from the group consisting of: about 135 µm, about 100 µm, about 60 µm, and about 50 µm.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
FIGS. 1A and 1B are diagrammatic schematic views of a medical sensing system according to some embodiments of the present disclosure.
FIG. 2 is a flow diagram of a method of performing a diagnostic procedure using the medical sensing system according to embodiments of the present disclosure.
FIG. 3 is a top view of a portion of an ultrasound scanner assembly according to an embodiment of the present disclosure.
FIG. 4 is a cross-sectional view of a transducer region of an ultrasound scanner assembly according to an embodiment of the present disclosure.
FIG. 5 is a further cross-sectional view of a transducer region of an ultrasound scanner assembly according to an embodiment of the present disclosure.
FIG. 6 is a longitudinal cross-sectional view of a rotational ultrasound scanner assembly according to an embodiment of the present disclosure.

### DETAILED DESCRIPTION

For the purposes of promoting an understanding of the principles of the present disclosure, reference will now be made to the implementations illustrated in the drawings and specific language will be used to describe them. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one or more implementations may be combined with the features, components, and/or steps described with respect to other implementations of the present disclosure. For simplicity, in some instances the same reference numbers are used throughout the drawings to refer to the same or like parts.

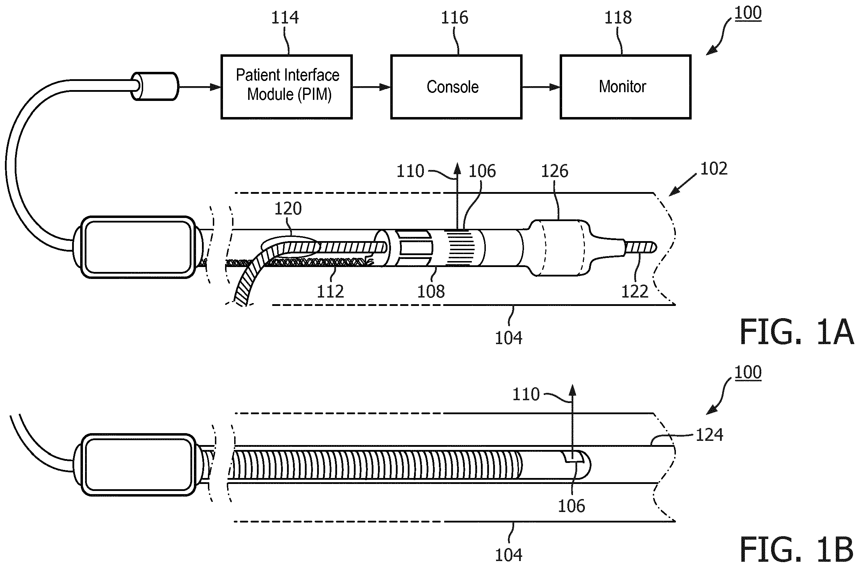

Some embodiments of the present disclosure relate generally to intravascular imaging devices and medical sensing systems that include one or more acoustic impedance matching structures. Referring to FIG. 1A, shown therein is a diagrammatic schematic view of a medical sensing system 100 according to an embodiment of the present disclosure. The medical sensing system 100 includes an elongate member 102 (such as a catheter, guide wire, or guide catheter) of the medical sensing system 100. As used herein, "elongate member" or "flexible elongate member" includes at least any thin, long, flexible structure that can be inserted into the vasculature of a patient. Flexible elongate members 102 include, for example, guide wires, catheters, and guide catheters. In that regard, a catheter may or may not include a lumen extending along its length for receiving and/or guiding other instruments. If the catheter includes a lumen, the lumen may be centered or offset with respect to the cross-sectional profile of the device. While the illustrated embodiments of the "elongate members" of the present disclosure have a cylindrical profile with a circular cross-sectional profile that defines an outer diameter of the flexible elongate member, in other instances, all or a portion of the flexible elongate members may have other geometric cross-sectional profiles (*e.g.*, oval, rectangular, square, elliptical, etc.) or an irregular cross-sectional profile.

The medical sensing system 100 may be utilized in a variety of applications and can be used to assess vessels and structures within a living body. To do so, the elongate member 102 is advanced into a vessel 104. Vessel 104 represents fluid filled or surrounded structures, both natural and man-made, within a living body that may be imaged and can include for example, but without limitation, cardiovascular vessels, valves within the blood or other bodily systems, and structures such as: organs including the liver, heart, and kidneys. In addition to imaging natural structures, the elongate member 102 may be used to image man-made structures such as, but without limitation, artificial heart valves, stents, shunts, filters, and other devices positioned within the body. The elongate member 102 includes sensors 106 disposed along the length of the member 102 to collect diagnostic data regarding the vessel 104. In various embodiments, the sensors 106 correspond to sensing modalities such as flow, optical flow, IVUS, photoacoustic IVUS, FL-IVUS, pressure, optical pressure, fractional flow reserve (FFR) determination, coronary flow reserve (CFR) determination, OCT, transesophageal echocardiography, image-guided therapy, other suitable modalities, and/or combinations thereof.

In the exemplary embodiment of FIG. 1A, the elongate member 102 includes a solid-state IVUS device, and the sensors 106 include an array of IVUS ultrasound transducers. The sensors 106 and their associated control circuitry may be incorporated into a scanner assembly 108. When the scanner assembly 108 is positioned near the area to be imaged, the control circuitry selects some of the IVUS transceivers to transmit an ultrasound pulse that is reflected by the vessel 104 and the surrounding structures. The sensors 106 may be arranged around the circumference of the elongate member 102 and positioned to emit ultrasound energy radially 110 in order to obtain a cross-sectional representation of the vessel 104 and the surrounding anatomy. The control circuitry also selects some transceivers to receive the echo signal. By stepping through sequences of transmit-receive sets, the medical sensing system 100 system can synthesize the effect of a mechanically scanned transducer element without moving parts.

FIG. 1B is a schematic view of a system that includes an alternative elongate member 102 according to some embodiments of the present disclosure. The elongate member 102 of FIG. 1B is typical of a rotational device such as a rotational IVUS ultrasound system, and the sensor 106 includes one or more IVUS transducers arranged to emit ultrasound energy in a radial direction 110. In such an embodiment, the sensor(s) 106 may be mechanically rotated around a longitudinal axis of the elongate member 102 to obtain a cross-sectional representation of the vessel 104.

Data from the sensor(s) 106 is transmitted via a cable 112 to a Patient Interface Module (PIM) 114 and/or a console 116. The PIM 114 is an isolation device as, in various surgical settings, patient safety requirements mandate physical and electrical isolation of the patient. Thus, if complete electrical isolation is required, the PIM 114 and the console 116 may be communicatively coupled by an optical, RF, or other non-conductive link. In less stringent environments, conductive communication links and/or power couplings may extend between the two. Moreover, in some embodiments, the PIM 114 and console 116 are collocated and/or part of the same system, unit, chassis, or module. Together the PIM 114 and console 116 assemble, process, and render the sensor data for display as an image on a monitor 118. For example, in various embodiments, the PIM 114 and/or the console 116 generates control signals to configure the sensor 106, generates signals to activate the sensor 106, performs amplification, filtering, and/or aggregating of sensor data, and formats the sensor data as an image for display. The allocation of these tasks and others between the PIM 114 and the console 116 is merely arbitrary.

A guide wire or guide catheter may be used to advance and withdrawn the elongate member 102. Accordingly, in some embodiments, the elongate member 102 includes a guide wire exit port 120 as shown in FIG. 1A. The guide wire exit port 120 allows a guide wire 122 to be inserted towards the distal end in order to direct the member 102 through a vascular structure (*e.g*., vessel 104). In some such embodiments, the elongate member 102 is a rapid-exchange catheter. Additionally or in the alternative, the elongate member 102 is advanced through the vessel 104 inside a guide catheter 124 as shown in FIG. 1B.

In some embodiments, the elongate member 102 includes an inflatable balloon portion 126 near the distal tip. The balloon portion 126 is open to a lumen that travels along the length of the IVUS device and ends in an inflation port (not shown). The balloon 126 may be selectively inflated and deflated via the inflation port.

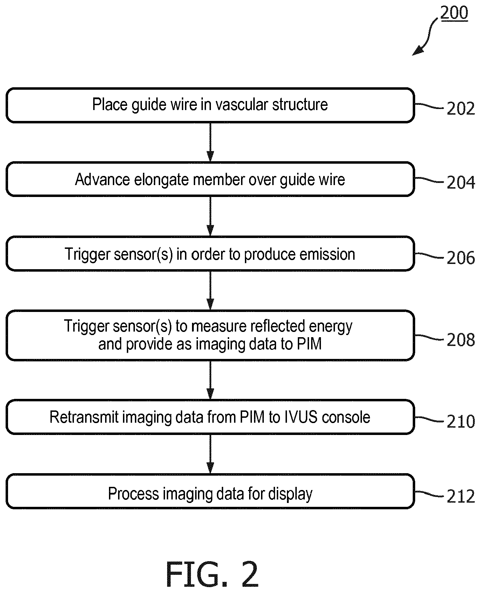

FIG. 2 is a flow diagram of a method 200 of performing a diagnostic procedure using the medical sensing system 100 according to embodiments of the present disclosure. It is understood that additional steps can be provided before, during, and after the steps of method 200, and that some of the steps described can be replaced or eliminated for other embodiments of the method.

Referring block 202 of FIG. 2 and referring still to FIGs. 1A and 1B, in an illustrative example of a typical environment and application of the system, a surgeon places a guide wire 122 in the vessel 104. The guide wire 122 is threaded through at least a portion of the distal end of the elongate member 102 before, during, or after placement of the guide wire 122. Referring to block 204 of FIG. 2, once the guide wire 122 is in place, the elongate member 102 is advanced over the guide wire. Additionally or in the alternative, a guide catheter 124 is advanced in the vessel 104 in block 202 and the elongate member 102 is advanced within the guide catheter in block 204.

Referring to block 206, once positioned, the sensor 106 is activated. Signals sent from the PIM 114 to the sensor 106 via the cable 112 cause the sensor to obtain diagnostic information. In the example of an IVUS application, transducers within the sensor 106 emit a specified ultrasonic waveform. The ultrasonic waveform is reflected by the vessel 104 and the surrounding anatomy. The reflections are received by the transducers and are amplified for transmission via the cable 112. The echo data is placed on the cable 112 and sent to the PIM 114.

In these examples and others, the PIM 114 may perform any suitable signal processing or enhancement before retransmitting the sensor data to the console 116 in block 208. Referring to block 210, the console 116 aggregates and assembles the received sensor data to create an image of the vessel 104 for display on the monitor 118. In some exemplary applications, the elongate member 102 is advanced beyond the area of the vessel 104 to be imaged and pulled back as the scanner assembly 108 is operating, thereby exposing and imaging a longitudinal portion of the vessel 104. To ensure a constant velocity, a pullback mechanism is used in some instances. A typical withdraw velocity is 0.5 cm/s. In some embodiments, the member 102 includes an inflatable balloon portion 126. As part of a treatment procedure, the balloon 126 may be positioned adjacent to a stenosis (narrow segment) or an obstructing plaque within the vessel 104 and inflated in an attempt to widen the restricted area of the vessel 104.

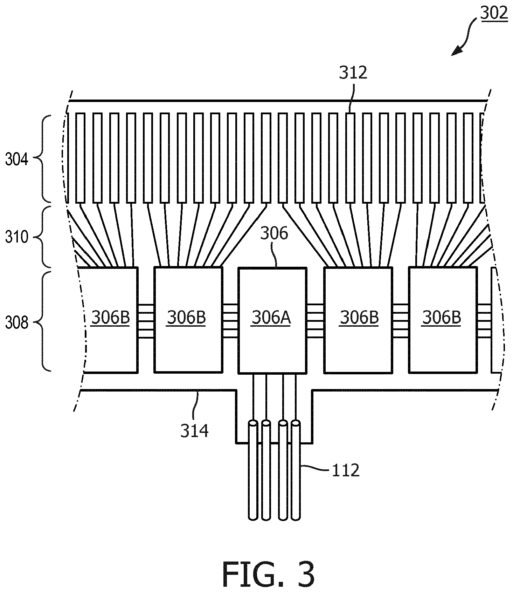

Various elongate members 102 and sensors 106 for both solid-state and rotational systems will now be described. Referring first to FIG. 3, shown is a solid-state ultrasound scanner assembly 302 suitable for use in the elongate member 102 such as that of FIG. 1A. FIG. 3 depicts the ultrasound scanner assembly 302 in its flat form during fabrication. The assembly 302 includes a transducer array 302 formed in a transducer region 304 and transducer control logic dies 306 (including dies 306A and 306B) formed in a control region 308, with a transition region 310 disposed therebetween. With respect to the transducer array 302, the array 302 may include any number and type of ultrasound transducers 312, although for clarity only a limited number of ultrasound transducers are illustrated in FIG. 3. In an embodiment, the transducer array 302 includes 64 individual ultrasound transducers 312. In a further embodiment, the transducer array 302 includes 32 ultrasound transducers 312. Other numbers of transducers are both contemplated and provided for. With respect to the types of transducers, in an embodiment, the ultrasound transducers 312 are piezoelectric micromachined ultrasound transducers (PMUTs) fabricated on a microelectromechanical system (MEMS) substrate using a polymer piezoelectric material, for example as disclosed in U.S. Patent 6,641,540. In alternate embodiments, the transducer array includes piezoelectric transducers fabricated from bulk PZT ceramic or single crystal piezoelectric material, capacitive micromachined ultrasound transducers (CMUTs), other suitable ultrasound transmitters and receivers, and/or combinations thereof.

The scanner assembly 302 may include various transducer control logic, which in the illustrated embodiment is divided into discrete control logic dies 306. In various examples, the control logic of the scanner assembly 302 performs: decoding control signals sent by the PIM 108 across the cable 114, driving one or more transducers 312 to emit an ultrasonic signal, selecting one or more transducers 312 to receive a reflected echo of the ultrasonic signal, amplifying a signal representing the received echo, and/or transmitting the signal to the PIM across the cable 114. In the illustrated embodiment, a scanner assembly 302 having 64 ultrasound transducers 312 divides the control logic across nine control logic dies 306, of which five are shown. Designs incorporating other numbers of control logic dies 306 including 8, 9, 16, 17 and more are utilized in other embodiments, and the control logic dies 306 are not necessarily homogenous. In general, the control logic dies 306 are characterized by the number of transducers they are capable of driving, and exemplary control logic dies 306 drive 4, 8, and 16 transducers.

The transducer control logic dies 306 and the transducers 312 are mounted on a flex circuit 314 that provides structural support and interconnects for electrical coupling. The flex circuit 314 may be constructed to include a film layer of a flexible polyimide or other polymer material such as KAPTON™ (trademark of DuPont). Other suitable materials include polyester films, other polyimide films, polyethylene napthalate films, or polyetherimide films, other flexible printed semiconductor substrates as well as products such as Upilex® (registered trademark of Ube Industries) and TEFLON® (registered trademark of E.I. du Pont). The film layer is configured to be wrapped around a ferrule to form a cylindrical toroid in some instances. Therefore, the thickness of the film layer is generally related to the degree of curvature in the final assembled scanner assembly 302. In some embodiments, the film layer is between 5 µm and 100 µm, with some particular embodiments being between 12.7 µm and 25.1 µm.

To electrically interconnect the control logic dies 306 and the transducers, in an embodiment, the flex circuit 314 further includes conductive traces formed on the film layer that carry signals between the control logic dies 306 and the transducers 312 and that provide a set of pads for connecting the conductors of cable 114. Suitable materials for the conductive traces include copper, gold, platinum, aluminum, silver, nickel, and tin and may be deposited on the flex circuit 314 by processes such as sputtering, plating, and etching. In an embodiment, the flex circuit 314 includes a chromium adhesion layer or a titanium-tungsten adhesion layer. The width and thickness of the conductive traces are selected to provide proper conductivity and resilience when the flex circuit 314 is rolled. In that regard, an exemplary range for the width of a conductive trace is between 10-50 µm. For example, in an embodiment, 20 µm conductive traces are separated by 20 µm of space. The width of a conductive trace may be further determined by the size of a pad of a device or the width of a wire to be coupled to the trace. The thickness of the conductive traces may have a range from about 1 µm to about 10 µm, with a typical thickness of 5 µm.

In some instances, the control logic dies 306 and the transducers 312 are mounted to the flex circuit 314 and then the scanner assembly 302 is transitioned from a flat configuration to a rolled (*i.e*., more cylindrical) configuration. For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND TRANSDUCER ASSEMBLY HAVING A FLEXIBLE SUBSTRATE,".

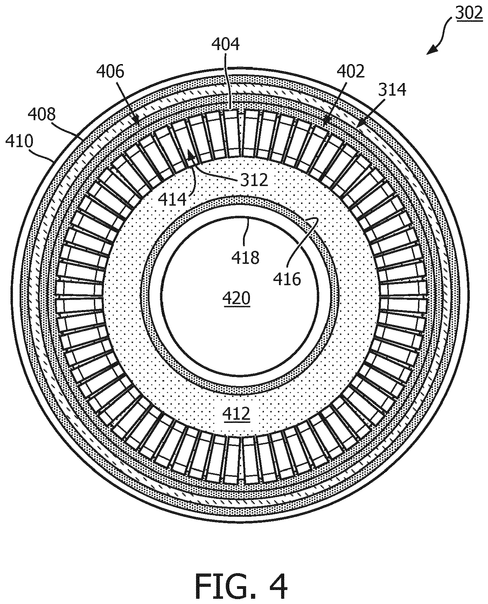

The rolled form of the ultrasound scanner is illustrated in FIG. 4. In that regard, FIG. 4 is a cross-sectional view of the transducer region 304 of the ultrasound scanner assembly 302 according to an embodiment of the present disclosure. As the name implies, the transducer region 304 of the scanner contains the transducers 312, which are physically attached to the flex circuit 314 by an underfill material 402 such as an epoxy or other adhesive. In some embodiments, the scanner assembly 302 includes one or more conductive layers (*e.g*., conductive layers 404-408) disposed on the transducers 312 and on the flex circuit 314. For example, conductive layer 404 is disposed directly on a transducer 312 between the transducer 312 and the flex circuit 314. Exemplary conductive layer 406 is disposed directly on the flex circuit 314 between the flex circuit 314 and the transducers 312. Exemplary conductive layer 408 is disposed directly on the flex circuit 314 opposite the transducers 312. The conductive layers 404-408 may include signal traces for electrically coupling the transducers 312, power traces for carrying supply voltage or ground, ground shielding, radiopaque markers, or other structures and may also be part of an impedance matching structure. Accordingly, the conductive layers 404-408 may include any suitable conductor such as gold, platinum, copper, aluminum, silver, nickel, and tin, and both the conductor and its physical dimensions may be configured to provide a desired acoustic interface.

Various examples will now be described. In an embodiment, the first conductive layer 404 is a connector of the respective transducer 312 and includes gold-containing conductors arranged in power traces for carrying supply voltage and/or ground signals. In some embodiments, the second conductive layer 406 includes gold and has any suitable thickness, measured radially, with some exemplary thicknesses being between about 0.5 µm and about 2.0 µm (*e.g*., about 0.5 µm, about 1.0 µm, about 1.5 µm, about 2.0 µm, etc.). In some embodiments, the third conductive layer 408 includes gold and has any suitable thickness, measured radially, with some exemplary thicknesses being between about 0.5 µm and about 1.0 µm (*e.g*., about 0.5 µm, about 1.0 µm, etc.). It is understood that various embodiments of the ultrasound scanner assembly 302 include some, all, or none of these conductive layers 404-408.

In the illustrated embodiment, the scanner assembly 302 also includes an outer membrane 410 used to insulate and cover the scanner assembly 302 and to protect the scanner assembly 302 from the environment. Insulator materials for the outer membrane 410 may be selected for their biocompatibility, durability, hydrophilic or hydrophobic properties, low-friction properties, ultrasonic permeability, and/or other suitable criteria. For example, the outer membrane 410 may include KAPTON™, polyester films, polyimide films, polyethylene napthalate films, Upilex®, Parylene™, heat shrink tubing such as polyester or PVDF, a melt-formable layers such as Pebax® (registered trademark of Arkema) or polyethylene, and/or other suitable membrane materials.

A backing material 412 may be applied to the transducers 312 to assist in the rolling of the scanner assembly 302 and to provide structure and rigidity. The backing material 412 may include any suitable material, such as an epoxy, and the composition may be selected to control the acoustic properties of the scanner assembly. For example, a ceramic material and/or a metal may be added to the epoxy to modify the acoustic impedance. Exemplary thicknesses for the backing material 412 range between about 135 µm and about 50 µm (*e.g*., about 135 µm, about 100 µm, about 60 µm, and about 50 µm) measured from the radial-most point of the transducers 312.

The scanner assembly may include inner conductive layers 414 and 416, each of which may include signal traces for electrically coupling the transducers 312, power traces for carrying supply voltage or ground, ground shielding, radiopaque markers, or other structures and may also be part of an impedance matching structure. The inner conductive layers 414 and 416 may include any suitable conductor such as gold, platinum, copper, aluminum, silver, nickel, and tin. For example, conductive layer 414 is disposed directly on the transducer 312 opposite the flex circuit 314 and may include gold-containing conductors arranged in power traces for carrying supply voltage and/or ground signals. Exemplary conductive layer 416 is disposed directly on the backing material 412 opposite the transducer 312 and contains platinum arranged to define a radiopaque marker. It is understood that various embodiments of the ultrasound scanner assembly 302 include one, both, or neither of these conductive layers 414 and 416.

In many embodiments, the flex circuit 314 and the attached elements are rolled around a ferrule 418. The lumen region 420 inside the ferrule 418 is open to allow the scanner assembly 302 to be advanced over a guide wire (not shown). The ferrule 418 may include a radiopaque material to aid in visualizing the scanner assembly 302 during a procedure.

As will be described in more detail below, any of the conductive layers 404, 406, 408, 414, and 416, the flex circuit 314, underfill material 402, the outer membrane 410, or the backing material 412 may be configured to provide an ultrasound interface tailored to improve the transmission of energy from the transducers 312 to the surrounding environment and to control ultrasonic reflections. Thus, a target ultrasonic response may determine the compositions, dimensions, constructions, and arrangement of these elements of the scanner assembly 302.

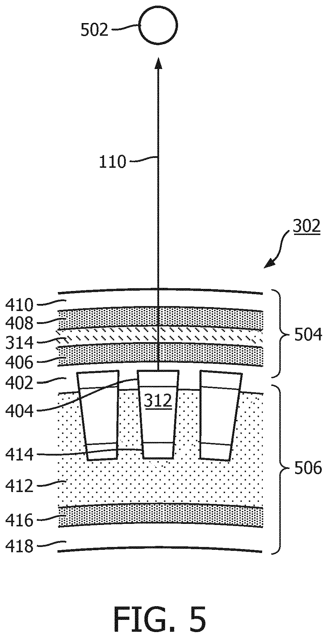

A portion of the scanner assembly 302 is shown and enlarged in FIG. 5, which is a cross-sectional view of the transducer region 304 of the ultrasound scanner assembly 302 according to an embodiment of the present disclosure. In particular, FIG. 5 shows the operation of the transducers 312 and shows some exemplary layers of the scanner assembly 302 that may be utilized as acoustic impedance matching structures to improve the imaging performance of the medical sensing system 100.

During imaging, some transducers 312 of the scanner assembly 302 will emit an ultrasound waveform, while some transducers 312 of the scanner assembly will listen for echoes produced by the waveform. Transducers 312 may operate as both emitters and receivers during the same firing. More than one transducer 312 may be activated concurrently in order to produce the ultrasound waveform. Firing transducers as a group may create a stronger ultrasonic transmission. Particularly in, but not limited to, embodiments using relatively small emitting transducers and/or embodiments imaging relatively long distances, a stronger emission improves the signal-to-noise ratio. Similarly, in some embodiments, a plurality of receiving transducers is set to receive as a group. The group of transducers may produce a stronger electrical potential with a better imaging characteristics than individual transducers acting alone.

The transmitting transducers 312 may be structured so that the ultrasound energy is generally directed radially outward as represented by arrow 110. For forward-looking embodiments, the beam may also be oriented out of the cross-sectional plane towards the distal tip of the elongate member 102. A portion of this ultrasonic energy is reflected by a target 502 located in the environment surrounding the scanner assembly 302 and returns to a receiving transducer 312, where it is measured. However, another portion of the outward-directed ultrasonic energy is reflected by structures within the scanner assembly 302 and is measured by the receiving transducer(s) 312 as noise. Similarly, a portion of the ultrasonic energy returning from the target 502 is reflected by structures within the scanner assembly 302 and never reaches the receiving transducer 312. Furthermore, in many embodiments, the ultrasound wavefront is not limited to just the desired narrow-width beam. Some portion of the ultrasound wavefront may be directed azimuthally as well as radially inward. This ancillary emission may also be reflected by structures within the scanner assembly 302. Reflections that constructively interfere with the outwardly-directed signal may increase transmission power and actually improve imaging performance. On the other hand, reflections that destructively interfere may create noise and obscure meaningful data.

To reduce the incidence of reflections and to improve propagation from the transducer 312 to the environment, the scanner assembly 302 may include one or more impedance matching structures. The structures may incorporate the various materials and layers of the scanner assembly 302 as well as dedicated acoustical materials. In the illustrated embodiment, the scanner assembly 302 includes a frontside impedance matching structure 504 that includes one or more of: the conductive layers 404-408, the flex circuit 314, the underfill material 402, and the outer membrane 410. In that regard, the respective materials, thicknesses, acoustic impedances (K), compositions, arrangement and/or other properties of these elements and others may be selected to such that the frontside impedance matching structure 504 provides an acoustic transition from the transducer 312 to the environment.

The characteristic acoustic impedance of the transducer 312 material, e.g., PZT, may be significantly different from the environment. In one example, the transducer 312 has an impedance of about 34 MRayl while water, blood and most biological tissue of interest in ultrasonic imaging has an acoustic impedance between about 1.5 MRayl to about 1.6 MRayl. In such an example, the frontside impedance matching structure 504 is configured to have an acoustic impedance between that of the transducer 312 and the environment. For example, the frontside impedance matching structure 504 may be configured to have an acoustic impedance that is substantially equal to a geometric mean of the transducer 312 impedance and the environment impedance. In this way, the frontside impedance matching structure 504 may be tuned to improve sensitivity, increase transmission strength, control reflections, increase bandwidth, attenuate frequencies outside the imaging frequency, and/or produce any other suitable effect.

In one such example, the frontside impedance matching structure 504 includes a conductive layer 406 substantially as described above and configured accordingly. In the example, the conductive layer 406 includes gold although any other suitable material may be used and may be formed to any suitable thickness such as about 0.5 µm, about 1.0 µm, about 1.5 µm, or about 2.0 µm. In a further example, the frontside impedance matching structure 504 includes a conductive layer 408 substantially as described above. In this example, the conductive layer 408 includes gold or other suitable material formed to any suitable thickness such as about 0.5 µm, or about 1.0 µm. In yet a further example, the frontside impedance matching structure includes both conductive layer 406 having a thickness between about 0.5 µm and about 2.0 µm and conductive layer 408 having a thickness between about 0.5 µm and about 1.0 µm.

Additionally or in the alternative, the scanner assembly 302 may include a backside impedance matching structure 506 made up of a combination of the backing material 412, and/or one or more of inner conductive layers 406 and 408. Similar to the frontside impedance matching structure 504, the backside impedance matching structure 506 may be tuned to improve sensitivity, increase transmission strength, control reflections, increase bandwidth, attenuate frequencies outside the imaging frequency, and/or produce any other suitable effect. In one example, the backside impedance matching structure 506 includes a backing material 412 being acoustically absorptive and having an impedance similar to that of the transducer 312. The backside impedance matching structure 506 may include an epoxy loaded with various materials in order to achieve a target impedance. For example, ceramic-loaded epoxy may have an acoustic impedance between about 3 MRayl and about 11 MRayl. Tungsten-loaded epoxy may have an acoustic impedance between about 6 MRayl and about 36 MRayl. The thickness of the backing material 412 may also be configured to control acoustic reflections, with exemplary thicknesses for the backing material 412 ranging from about 135 µm to about 50 µm (e.g., about 135 µm, about 100 µm, about 60 µm, about 50 µm, etc.).

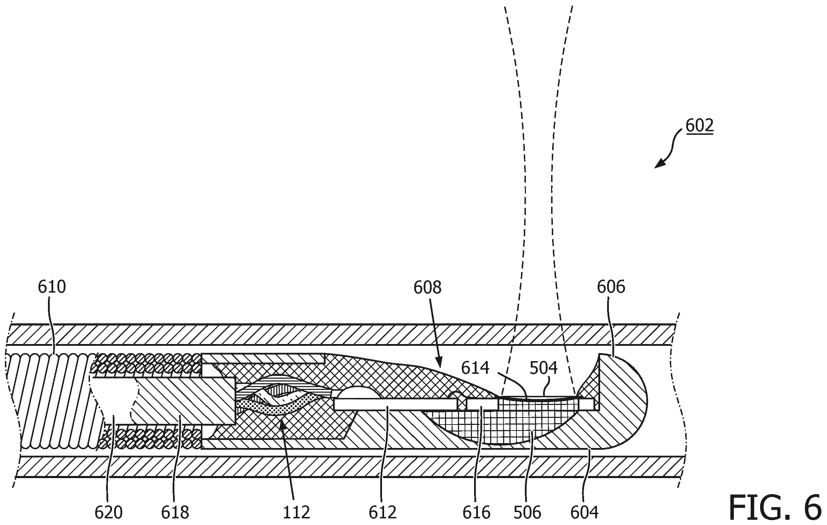

Impedance matching structures are equally applicable to rotational devices, and one such rotational ultrasound scanner assembly 602 suitable for use in an elongate member 102, such as that of FIG. 1B, is shown in FIG. 6. In that regard, FIG. 6 is a longitudinal cross-sectional view of a rotational ultrasound scanner assembly 602 according to an embodiment of the present disclosure. In this exemplary embodiment, the scanner assembly 602 is terminated at its distal tip by a housing 604 fabricated from stainless steel and provided with a rounded nose 606 and a cutout 608 for the ultrasound beam to emerge from the housing 604. In some embodiments, a flexible driveshaft 610 attached to the scanner assembly 602 is composed of two or more layers of counter wound stainless steel wires, welded, or otherwise secured to the housing 604 such that rotation of the flexible driveshaft 610 also imparts rotation on the housing 604.

The scanner assembly 602 may include a control circuit 612 (*e.g*., an ASIC, a µ-controller, etc.) and a transducer 614 (*e.g*., a PMUT MEMS transducer). In turn, the control circuit 612 may include an amplifier, a transmitter, and a protection circuit associated with the transducer 614. In the illustrated embodiment, the control circuit 612 and the transducer 614 are wire-bonded and glued together to form an ASIC/MEMS hybrid assembly, which is mounted to the transducer housing 616 and secured in place with epoxy. In other embodiments, the control circuit 612 and the transducer 614 are flip-chip mounted to the substrate of the transducer 614 using anisotropic conductive adhesive or suitable alternative chip-to-chip bonding method. In still other embodiments, both the control circuit 612 and the transducer 614 are attached to a flexible circuit substrate, which includes conductive paths to electrically connect the two components. In these embodiments and others, the leads of the multi-conductor electrical cable 112 with optional shield 618 and jacket 620 are soldered or otherwise electrically coupled directly to the control circuit 612. The electrical cable 112 extends through an inner lumen of the flexible driveshaft 610 to the proximal end of the scanner assembly 612 where it is terminated to the electrical connector portion of the rotational interface.

Similar to the preceding synthetic aperture examples, the scanner assembly 602 may include a frontside impedance matching structure 504 and/or a backside impedance matching structure 506 disposed on the transducer 614, and structured substantially as described above.

Persons skilled in the art will also recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An imaging device, comprising:
a flexible elongate member (102);
an ultrasound transducer (312) disposed at a distal portion of the flexible elongate member (102) and oriented to produce an ultrasound emission in a first direction (110); and
an acoustic impedance matching structure (504) coupled to the ultrasound transducer (312); wherein the acoustic impedance matching structure (504) is disposed radially outward from the ultrasound transducer (312) and includes:
a polymer film (314);
a first conductor layer (408) disposed radially outward on the polymer film (314), and
a second conductor layer (406) disposed radially inward on the polymer film (314);
wherein the first conductor layer (408) and the second conductor layer (406) are both configured to provide ground shielding.

2. The imaging device of claim 1, wherein a third conductor (404) layer is disposed on the ultrasound transducer (312) and an underfill material (402) is disposed between the third conductor layer (404) and the second conductor layer (406).

3. The imaging device of claim 1, wherein the second conductor layer (406) has a thickness in a radial direction (110) between about 0.5 µm and about 2.0 µm.

4. The imaging device of claim 1, wherein the first conductor layer (408) has a thickness in a radial direction between about 0.5 µm and about 1.0 µm.

5. The imaging device of claim 1, wherein the first and second conductor layers (408, 406) include gold.

6. The imaging device of claim 1 further comprising a backside acoustic impedance matching structure (506), the backside acoustic impedance matching structure (506) being disposed radially inward from the ultrasound transducer (312) and further including a backing material (412).

7. The imaging device of claim 6, wherein the backing material (412) includes an epoxy containing at least one of: a ceramic material or a metal.

8. The imaging device of claim 7, wherein the backing material has a thickness in a radial direction between about 135 µm and about 50 µm.

9. The imaging device according to claim 1, further comprising
additional ultrasound transducers (312) disposed at the distal portion of the flexible elongate member the ultrasound transducer (312) and the additional ultrasound transducers (312) being a plurality of ultrasound transducers arranged circumferentially to form an ultrasound scanner assembly (108, 302); and
wherein the impedance matching structure (504) is disposed radially outward from the plurality of ultrasound transducers (312).

10. The imaging device of claim 9 further comprising a backing material (412) disposed radially inward from the plurality of ultrasound transducer (312).

11. The imaging device of claim 10, wherein the backing material (412) includes an epoxy and at least one of: a ceramic material or a metal.

## Patentansprüche

1. Bildgebungsvorrichtung, umfassend:
ein flexibles längliches Element (102);
einen Ultraschallwandler (312), der an einem distalen Abschnitt des flexiblen länglichen Elements (102) angeordnet ist und ausgerichtet ist, um eine Ultraschallemission in eine erste Richtung (110) zu erzeugen; und
eine Akustische-Impedanz-Anpassungs-Struktur (504), die mit dem Ultraschallwandler (312) verbunden ist; wobei die Akustische-Impedanz-Anpassungs-Struktur (504) radial nach außen von dem Ultraschallwandler (312) angeordnet ist und Folgendes einschließt:
eine Polymerfolie (314),
eine erste Leiterschicht (408), die radial nach außen auf der Polymerfolie (314) angeordnet ist;
eine zweite Leiterschicht (406), die radial nach innen auf der Polymerfolie (314) angeordnet ist;
wobei die erste Leiterschicht (408) und die zweite Leiterschicht (406) beide konfiguriert sind, um eine Erdungsabschirmung bereitzustellen.

2. Bildgebungsvorrichtung nach Anspruch 1, wobei eine dritte Leiterschicht (404) auf dem Ultraschallwandler (312) angeordnet ist und ein Underfill-Material (402) zwischen der dritten Leiterschicht (404) und der zweiten Leiterschicht (406) angeordnet ist.

3. Bildgebungsvorrichtung nach Anspruch 1, wobei die zweite Leiterschicht (406) eine Dicke in eine radiale Richtung (110) zwischen etwa 0,5 µm und etwa 2,0 µm aufweist.

4. Bildgebungsvorrichtung nach Anspruch 1, wobei die erste Leiterschicht (408) eine Dicke in eine radiale Richtung zwischen etwa 0,5 µm und etwa 1,0 µm aufweist.

5. Bildgebungsvorrichtung nach Anspruch 1, wobei die erste und die zweite Leiterschicht (408, 406) Gold einschließen.

6. Bildgebungsvorrichtung nach Anspruch 1, weiter umfassend eine Rückseitige-akustische-Impedanz-Anpassungs-Struktur (506), wobei die Rückseitige-akustische-Impedanz-Anpassungs-Struktur (506) radial nach innen von dem Ultraschallwandler (312) angeordnet ist und weiter ein Trägermaterial (412) einschließt.

7. Bildgebungsvorrichtung nach Anspruch 6, wobei das Trägermaterial (412) ein Epoxid einschließt, das mindestens eines von einem Keramikmaterial oder einem Metall enthält.

8. Bildgebungsvorrichtung nach Anspruch 7, wobei das Trägermaterial eine Dicke in eine radiale Richtung zwischen etwa 135 µm und etwa 50 µm aufweist.

9. Bildgebungsvorrichtung nach Anspruch 1, weiter umfassend
zusätzliche Ultraschallwandler (312), die am distalen Abschnitt des flexiblen länglichen Elements angeordnet sind, wobei der Ultraschallwandler (312) und die zusätzlichen Ultraschallwandler (312) eine Vielzahl von Ultraschallwandlern sind, die umlaufend arrangiert sind, um eine Ultraschallscannerbaugruppe (108, 302) zu bilden; und
wobei die Impedanzanpassungsstruktur (504) radial nach außen von der Vielzahl von Ultraschallwandlern (312) angeordnet ist.

10. Bildgebungsvorrichtung nach Anspruch 9, weiter umfassend ein Trägermaterial (412), das radial nach innen von der Vielzahl von Ultraschallwandlern (312) angeordnet ist.

11. Bildgebungsvorrichtung nach Anspruch 10, wobei das Trägermaterial (412) ein Epoxid und mindestens eines von einem Keramikmaterial oder einem Metall einschließt.

## Revendications

1. Dispositif d'imagerie comprenant :
un élément allongé flexible (102) ;
un transducteur à ultrasons (312) disposé au niveau d'une partie distale de l'élément allongé flexible (102) et orienté pour produire une émission ultrasonore dans une première direction (110) ; et
une structure d'adaptation d'impédance acoustique (504) couplée au transducteur à ultrasons (312) ; dans lequel la structure d'adaptation d'impédance acoustique (504) est disposée radialement vers l'extérieur du transducteur à ultrasons (312) et inclut :
un film polymère (314) ;
une première couche conductrice (408) disposée radialement vers l'extérieur sur le film polymère (314), et
une deuxième couche conductrice (406) disposée radialement vers l'intérieur sur le film polymère (314) ;
dans lequel la première couche conductrice (408) et la deuxième couche conductrice (406) sont toutes deux configurées pour fournir un blindage à la terre.

2. Dispositif d'imagerie selon la revendication 1, dans lequel une troisième couche conductrice (404) est disposée sur le transducteur à ultrasons (312) et un matériau de remplissage (402) est disposé entre la troisième couche conductrice (404) et la deuxième couche conductrice (406).

3. Dispositif d'imagerie selon la revendication 1, dans lequel la deuxième couche conductrice (406) a une épaisseur dans une direction radiale (110) comprise entre environ 0,5 µm et environ 2,0 µm.

4. Dispositif d'imagerie selon la revendication 1, dans lequel la première couche conductrice (408) a une épaisseur dans une direction radiale comprise entre environ 0,5 µm et environ 1,0 µm.

5. Dispositif d'imagerie selon la revendication 1, dans lequel les première et deuxième couches conductrices (408, 406) incluent de l'or.

6. Dispositif d'imagerie selon la revendication 1, comprenant en outre une structure d'adaptation d'impédance acoustique postérieure (506), la structure d'adaptation d'impédance acoustique postérieure (506) étant disposée radialement vers l'intérieur du transducteur à ultrasons (312) et incluant en outre un matériau de renfort (412).

7. Dispositif d'imagerie selon la revendication 6, dans lequel le matériau de renfort (412) comprend un époxy contenant au moins l'un parmi : un matériau céramique ou un métal.

8. Dispositif d'imagerie selon la revendication 7, dans lequel le matériau de renfort a une épaisseur dans une direction radiale comprise entre environ 135 µm et environ 50 µm.

9. Dispositif d'imagerie selon la revendication 1, comprenant en outre
des transducteurs à ultrasons supplémentaires (312) disposés au niveau de la partie distale de l'élément allongé flexible, le transducteur à ultrasons (312) et les transducteurs à ultrasons supplémentaires (312) étant une pluralité de transducteurs à ultrasons agencés de manière circonférentielle pour former un ensemble de scanner à ultrasons (108, 302) ; et
dans lequel la structure d'adaptation d'impédance (504) est disposée radialement vers l'extérieur de la pluralité de transducteurs à ultrasons (312).

10. Dispositif d'imagerie selon la revendication 9, comprenant en outre un matériau de renfort (412) disposé radialement vers l'intérieur de la pluralité de transducteurs à ultrasons (312).

11. Dispositif d'imagerie selon la revendication 10, dans lequel le matériau de renfort (412) inclut un époxy et au moins l'un parmi : un matériau céramique ou un métal.
